# EUROPÄISCHE PATENTANMELDUNG

(11) **EP 0 806 656 A2**
(43) Veröffentlichungstag der Anmeldung: **12.11.1997**
(21) Anmeldenummer: 97106736.8
(22) Anmeldetag: 23.04.1997
(51) Int. Cl.: G01N 27/12

(54) **Gassensor und Verfahren zur Herstellung eines Gassensors**

(30) Priorität: 10.05.1996 DE 19618935
(71) Anmelder: SIEMENS AKTIENGESELLSCHAFT, 80333 München (DE)
(72) Erfinder: Seth, Michael, 35440 Linden (DE); Fleischer, Maximilian, Dr., 85635 Höhenkirchen (DE); Meixner, Hans, Prof.-Dr., 85540 Haar (DE)

(57) **Zusammenfassung**

Zur Erhöhung der Selektivität und der Sensitivität auf ein zu messendes Gas wird die gassensitive Galliumoxidschicht des Gassensors mit einer Filterschicht, die Siliziumdioxid aufweist, bedeckt. Alternativ kann die Galliumoxidschicht mit einer gassensitiven Schicht aus Titanoxid, Aluminiumvanadat, Wolframoxid oder Tantaloxid zu bedeckt sein.

## Beschreibung

Die Erfindung betrifft einen Gassensor auf der Basis eines Metalloxids zur Detektion eines Gases in einem Gasgemisch. Gassensoren auf der Basis eines Metalloxids verändern grundsätzlich ihre elektrische Leitfähigkeit in Abhängigkeit vom umgebenden Gas.

Bisher war es nur möglich, die gassensitiven Eigenschaften des Metalloxids durch die Veränderung der Betriebstemperatur zu beeinflußen und dadurch die Sensitivität auf bestimmte Gase zu vergrößern und auf andere Gase zu verringern. Hierzu wird auf die beiden Druckschriften EP 0 527 258 B1 und EP 0 464 243 B1 verwiesen. In der letztgenannten Druckschrift wird ein Sauerstoffsensor mit halbleitendem Galliumoxid beschrieben. Liegt die Betriebstemperatur des Gassensors über 850 °C, dann steht der Sauerstoffgehalt des Materials in Wechselwirkung mit dem Sauerstoffgehalt der Umgebungsatmosphäre, so daß die spezifische elektrische Leitfähigkeit des Materials ein Maß für den gegenwärtig herrschenden Sauerstoffpatialdruck ist. Soll ein Gassensor für reduzierende Gase geschaffen werden, so ist gemäß der EP 0 527 258 B1 die Betriebstemperatur für die Ga₂O₃-Dünnschicht bei ca. 600 °C festzulegen.

Aus dem Stand der Technik US 4 347 732 ist ein Gassensor zur Detektion bestimmter brennbarer und toxischer Gase bekannt. Der Gassensor weist eine gassensitive Schicht auf, die mit Galliumoxid dotiertes Zinkoxid enthält. Diese Schicht ist mit einer Filterschicht aus einem Zeolith, beispielsweise Zeolith 3 A, bedeckt. Der Gassensor wird bei einer Teperatur von 200°C betrieben. Für Temperaturen zwischen 600 und 1000°C ist der Gassensor nicht geeignet , da es in diesem Temperaturbereich zu einer Abdampfung der Zeolithschicht kommt.

Die Aufgabe der Erfindung ist es, einen Gassensor anzugeben, dessen Selektivität und dessen Sensitivität bezogen auf das zu messende Gas erhöht wird.

Eine Filterschicht, die Siliziumdioxid (SiO₂) aufweist hat den Vorteil, daß sie unempfindlich gegenüber aggressiven Gasen ist.

Die Aufgabe wird durch einen Gassensor gemaß den Ansprüchen 1 und 5 gelöst.

Vorteilhafte Weiterbildungen ergeben sich aus den Unteransprüchen.

So ist es von Vorteil, gemäß Anspruch 2 die Dicke der Filterschicht zwischen 300 und 500 nm zu wählen, da bei einer zu dünnen Filterschicht die Selektivität verschwindet und bei einer zu dicken Filterschicht die Sensitivität zu stark verringert wird.

Zur Verbesserung der Sensitivität des Gassensors auf H₂ hat die Betriebstemperatur, gemäß Anspruch 3, zwischen 600 °C und 700 °C zu liegen. Bei einer niedrigeren Temperatur ist der elektrische Widerstand der gassensitiven Schicht zu hoch. Bei einer höheren Temperatur geht die Sensitivität auf H₂ zurück.

Die Erfindung wird anhand mehrerer Figuren weiter erläutert.
- Figur 1: zeigt den Sensoraufbau in der Draufsicht und in der Unteransicht.
- Figur 2: zeigt vereinfacht dargestellt den Sensor im Querschnitt.
- Figur 3: zeigt Meßdiagramme, bei denen ein konventioneller Ga₂O₃-Sensor mit dem erfindungsgemäßen Gassensor verglichen wird.
- Figur 4: zeigt Meßdiagramme, bei denen wiederum ein konventioneller Ga₂O₃-Sensor mit dem erfindungsgemäßen Gassensor verglichen wird, jedoch gegenüber Figur 3, bei anderen Meßgasen.
- Figur 5: zeigt Meßdiagramme, bei denen ein bei 600 °C betriebener konventioneller Gassensor mit einem ebenfalls bei 600 °C betriebenen erfindungsgemäßen Gassensor, der mit einer Titanoxidschicht bedeckt ist, verglichen wird.
- Figur 6: zeigt Meßdiagramme, bei denen wiederum ein konventioneller Galliumoxid-Sensor mit dem erfindungsgemäßen Gassensor, der mit Titanoxid bedeckt ist, verglichen wird, jedoch gegenübeFigur 5, bei anderen Meßgasen.
- Figur 7: zeigt Meßdiagramme, bei denen ein konventioneller Galliumoxid-Sensor bei einer Betriebstemperatur von 700 °C mit einem ebenfalls bei 700 °C betriebenen erfindungsgemäßen Gassensor, der mit Tantaloxid bedeckt ist, verglichen wird.
- Figur 8: zeigt Meßdiagramme, bei denen wiederum ein konventioneller Galliumoxid-Sensor mit dem erfindungsgemäßen Gassensor, der mit Tantaloxid bedeckt ist, verglichen wird, jedoch gegenüber Figur 7, bei anderen Meßgasen.
- Figur 9: zeigt Meßdiagramme, bei denen ein konventioneller Galliumoxid-Sensor bei einer Betriebstemperatur von 700 °C mit einem ebenfalls bei 700 °C betriebenen erfindungsgemäßen Gassensor, der vor dem zweiten Tempervorgang mit Aluminiumvanadat bedeckt wurde, verglichen wird.
- Figur 10: zeigt Meßdiagramme, bei denen wiederum ein konventioneller Galliumoxid-Sensor mit dem erfindungsgemäßen Gassensor, der vor dem zweiten Tempervorgang mit Aluminiumvanadat bedeckt wurde, verglichen wirdjedoch gegenüber Figur 9, bei anderen Meßgasen.
- Figur 11: zeigt Meßdiagramme, bei denen ein konventioneller Galliumoxid-Sensor bei einer Betriebstemperatur von 600 °C mit einem erfindungsgemäßen Gassensor bei einer Betriebstemperatur von 500 °C, der vor dem zweiten Tempervorgang mit Aluminiumvanadat bedeckt wurde, verglichen wird.
- Figur 12: zeigt Meßdiagramme, bei denen wiederum ein konventioneller Galliumoxid-Sensor mit dem erfindungsgemäßen Gassensor, der vor dem zweiten Tempervorgang mit Aluminiumvanadat bedeckt wurde, verglichen wird, jedoch gegenüber Figur 11, bei anderen Meßgasen.
- Figur 13: zeigt Meßdiagramme, bei denen die Sensitivität eines erfindungsgemäßen Gassensors, der vor dem zweiten Tempervorgang mit Aluminiumvanadat bedeckt wurde, bei verschiedenen Betriebstemperaturen (700°C, 800 °C und 900 °C) bei verschiedenen Meßgasen.
- Figur 14: zeigt Meßdiagramme, bei denen ein konventioneller Galliumoxid-Sensor bei 900 °C Betriebstemperatur, mit einem erfindungsgemäßen Gassensor, der vor dem zweiten Tempervorgang mit Aluminiumvanadat bedeckt wurde, verglichen wird.
- Figur 15: zeigt Meßdiagramme, bei denen ein konventioneller Galliumoxid-Sensor bei einer Betriebstemperatur von 1000 °C mit einem ebenfalls bei 1000 °C betriebenen erfindungsgemäßen Gassensor, der vor dem zweiten Tempervorgang mit Aluminiumvanadat bedeckt wurde, verglichen wird.
- Figur 16: zeigt Meßdiagramme, bei denen ein konventioneller Galliumoxid-Sensor mit dem erfindungemäßen Sensor, der vor dem zweiten Tempervorgang mit Aluminiumvanadat bedeckt wurde, verglichen wird, jedoch gegenüber Figur 17, bei anderen Meßgasen.
- Figur 17: zeigt ein Meßdiagramm, bei dem die Sensitivität eines erfindungsgemäßen Gassensors dargestellt ist, der mit Wolframoxid bedeckt ist.

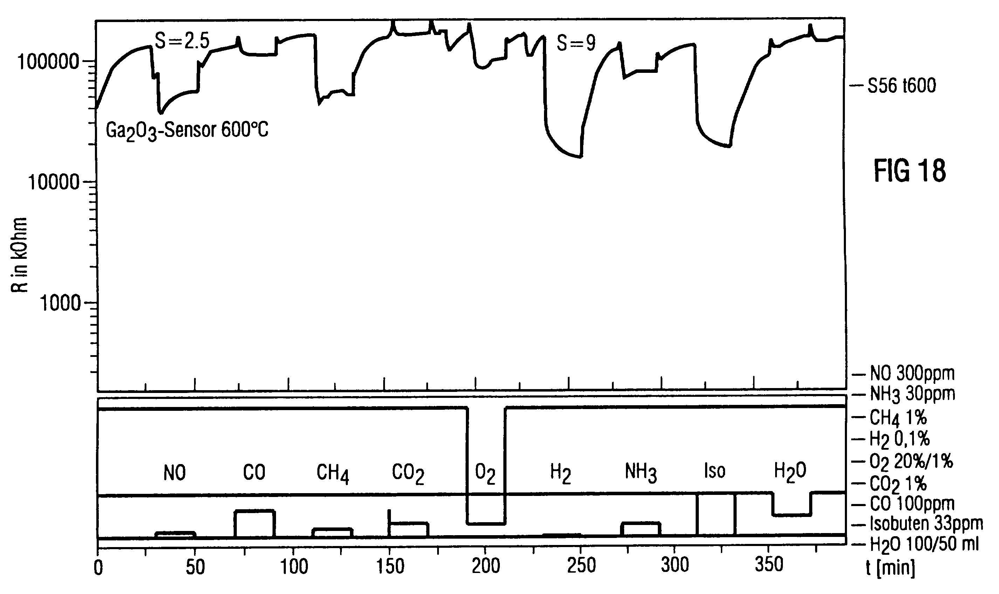

- Figur 18: zeigt ein Meßdiagramm, bei dem die Sensitivität eines konventionellen Galliumoxid-Sensors bei einer Betriebstemperatur von 600 °C dargestellt ist, wobei dieser denselben Meßbedingungen ausgesetzt ist, wie sie in Figur 15 gezeigt sind.

### 1. Ausführungsform:

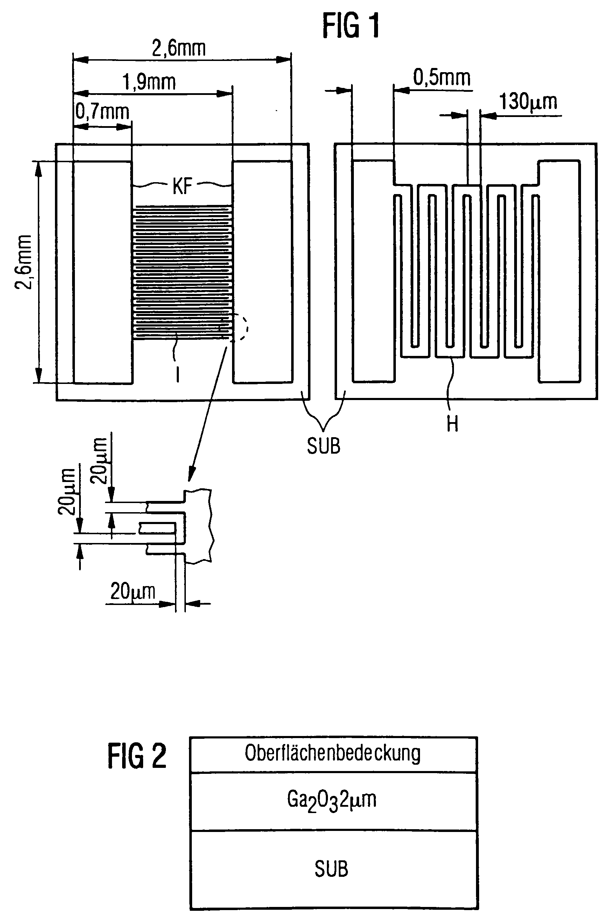

Fig. 1 links zeigt den Sensor in der Draufsicht. Auf einem Substrat SUB sind metallische Kontaktflächen KF vorgesehen, zwischen denen eine Interdigitalstruktur I angeordnet ist. Ein Ausschnitt der Interdigitalstruktur I ist in Fig. 1 unten vergrößert gezeigt. Über der Interdigitalstruktur I ist eine gassensitive Schicht aus Ga₂O₃ vorgesehen. Auf der Unterseite des Substrats SUB, in Fig. 1 rechts dargestellt, ist eine Heizanordnung H aufgebracht.

Figur 2 stellt den Sensor in stark vereinfachter Form im Querschnitt dar. Auf dem Substrat SUB ist eine 2 µm dicke Ga₂O₃-Schicht aufgebracht. Über dieser Schicht ist eine Filterschicht, auch als Oberflächenbedeckung bezeichnet, aus SiO₂ angeordnet. Die Dicke der Filterschicht beträgt etwa 300 nm. Der Einfachheit halber sind die Interdigitalstruktur I und die Heizanordnung H in Figur 1 nicht dargestellt.

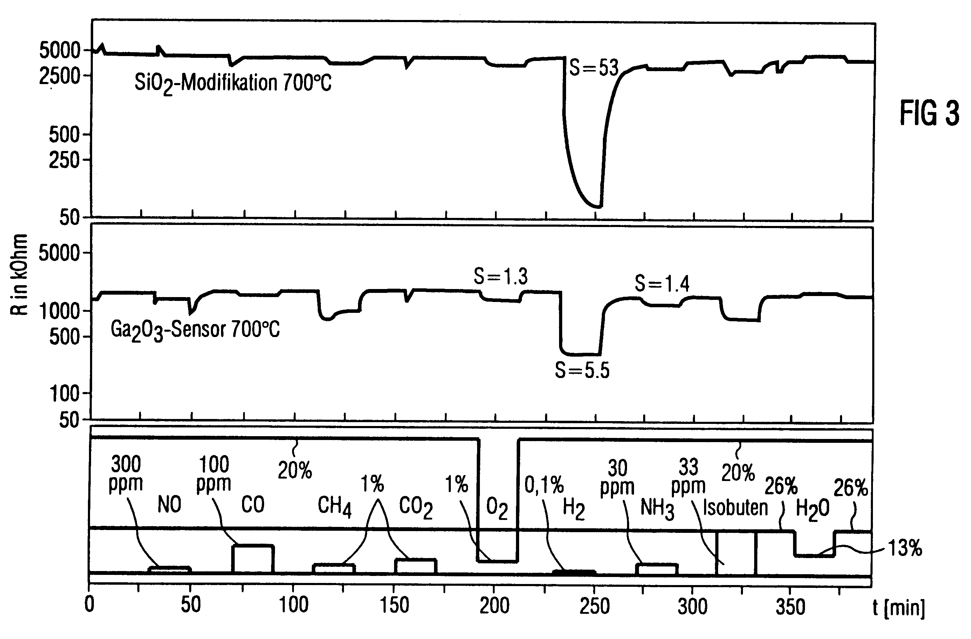

In Fig. 3 wird der erfindungsgemäße Gassensor einem konventionellen Sensor auf Ga₂O₃-Basis gegenübergestellt. Im unteren Teil der Fig. 3 ist über der Zeit t (in Minuten) aufgetragen, wie lange der Sensor welchem Gas ausgesetzt ist. Aus den beiden darüberliegenden Diagrammen können die Reaktionen des konventionellen Galliumoxid-Sensors und des mit SiO₂ modifizierten Sensors (= erfindungsgemäßer Sensor) bei dem jeweils vorherrschenden Meßgas entnommen werden. Auf der Ordinate der beiden Diagramme ist der Widerstand R in kOhm angetragen. Die im unteren Teil des Diagramms unter den jeweiligen Meßgasen angetragenen Balken stehen nicht im Verhältnis zueinander, sondern dienen lediglich der Kontrolle, ob der Gasfluß vorhanden und konstant war. Bis zum Zeitpunkt t = 350 min herrscht eine gleichbleibende Feuchtigkeit. Ab dem Zeitpunkt t = 350 min wird die relative Feuchtigkeit (= H₂0-Konzentration) von 26 % auf 13 % für ca. 20 min reduziert. Die Sauerstoffkonzentration wird zum Zeitpunkt t = 190 min für ca. 20 min von 20 % auf 1 % reduziert. D. h., es sind dann 98 % Stickstoff, 1 % H₂O und 1 % Sauerstoff im zu untersuchenden Gasgemisch vorhanden. Es wurden jeweils Konzentrationen gewählt, die bei einer technischen Anwendung zu erwarten sind. Man erkennt deutlich, daß die Sensitivität S für Wasserstoff (H₂) beim erfindungsgemäßen Gassensor erheblich höher ist, nämlich S = 53, als beim konventionellen Gassensor, nämlich S = 5,5. Auch die mangelnde Selektivität des konventionellen Gassensors ist beim Vergleich der beiden Diagramme feststellbar. Eine deutliche Querempfindlichkeit zeigt sich bei Sauerstoff (O₂), Ammoniak (NH₃), Methan (CH₄) und Isobuten. Die Sensitivität S ergibt sich aus dem Quotienten aus dem ohmschen Widerstand des Sensors in synthetischer Luft und dem ohmschen Widerstand des Sensors im Prüfgas.

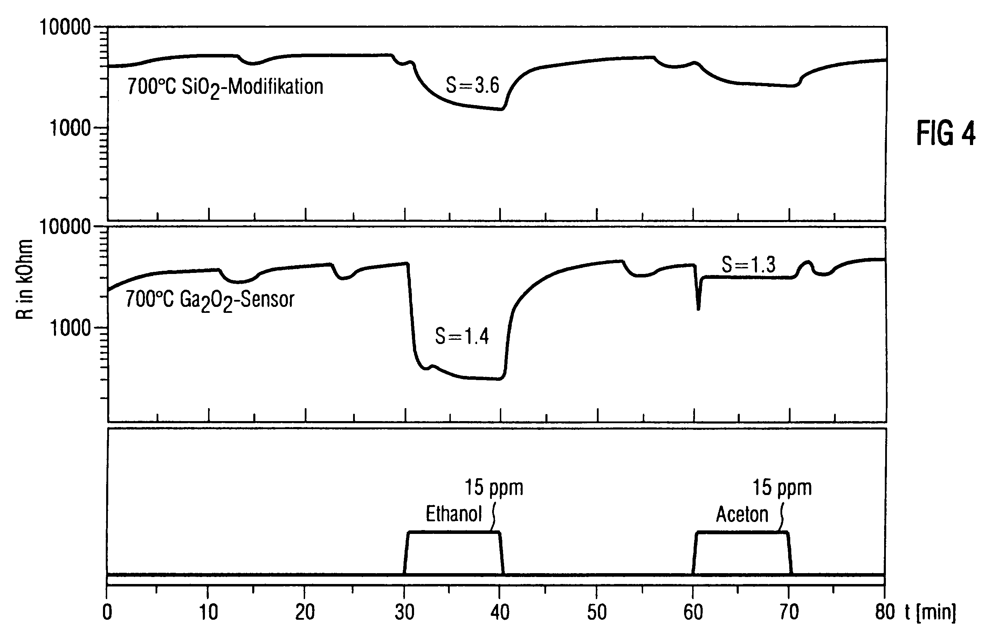

In Figur 4 sind ebenfalls die Änderungen der Sensitivitäten aufgetragen, gegenüber Figur 3 jedoch in Abhängigkeit von Ethanol und Aceton.

Der Vergleich des erfindungsgemäßen Sensors mit dem konventionellen Gassensor zeigt, daß durch die SiO₂-Oberflächenbedeckung die Methan-, Ethanol- und Isobutensensitivität verringert werden konnte, wohingegen die Wasserstoffsensitivität wesentlich angewachsen ist. Die SiO₂-Schicht ist nur für Wasserstoff permeabel. Das bedeutet, daß die Luftfeuchtigkeit von der gassensitiven Schicht abgehalten wird, wodurch der Widerstand des erfindungsgemäßen Sensors gegenüber dem konventionellen Sensor um einen Faktor 5 höher liegt (vgl. Fig. 3, ca. 1 MOhm beim konventionellen Gassensor und 5 MOhm beim erfindungsgemäßen Gassensor). Somit nimmt auch die Sensitivität auf Wasserstoff beim erfindungsgemäßen Sensor zu. Beide Sensoren wurden im Test bei einer Betriebstemperatur von 700 °C betrieben.

Der erfindungsgemäße Sensor kann vorteilhafterweise in kostengünstiger und mikrosystemkompatibler Planartechnologie verwirklicht werden. Vom Standpunkt der Reproduzierbarkeit der Herstellungstechnologie aus gesehen, ist die Planartechnologie gegenüber dem bulk-keramischen Aufbau vorzuziehen.

Bei der Herstellung sind folgende Schritte zu beachten:

Der Ga₂O₃-Sensor wird auf die bekannte Weise hergestellt, beispielsweise durch Abscheidung. Die Kristallinität und die Stöchiometrie der Ga₂O₃-Schicht lassen sich durch einen ersten Temperprozeß bei Temperaturen zwischen 700 und 1200 °C verbessern.

Die zusätzliche Schicht aus SiO₂ wird auf der Oberfläche des Ga₂O₃ einige Nanometer bis Mikrometer dick aufgebracht. Die Oberflächenmodifikation kann durch Kathodenzerstäubung (= Sputtern), CVD, Elektronenstrahlverdampfung, Molekularstrahlverfahren oder naßchemische Verfahren (Sol-Gel-Verfahren) erfolgen.

Durch einen geeigneten zweiten Temperprozeß wird diese Oberfläche stabilisiert.

Durch die Oberflächenbedeckung kann die Gassensitivität in hohem Maße beeinflußt werden. Die Sensitivitäten auf bestimmte Gase können vergrößert und die Querempfindlichkeiten auf störende Gase verringert oder zum Teil ganz beseitigt werden.

Die filternde Wirkung der SiO₂-Schicht beruht auf 2 Effekten:

Zum einen entscheidet die Porengröße der Schicht, welche Gasteilchen zum gassensitiven Material gelangen. Zum anderen ist der polare Charakter (hydrophil, hydrophob) der gasfilternden Schicht und der Gase ausschlaggebend. So ist es denkbar, daß nur polare oder unpolare Gasmoleküle, abhängig von dem polaren Zustand der Filterschicht diese passieren können (z. B. Feuchtigkeitssperre durch hydrophobe Membrane).
Das heißt, wenn das Gas und die Filterschicht unterschiedlichen polaren Zustand aufweisen, kann das Gas nicht durch die Filterschicht hindurchtreten.

Durch die filternde Schicht aus SiO₂ kann nicht nur die Selektivität, sondern auch die Sensitivität für bestimmte Gase verbessert werden. Dieses Verhalten läßt sich damit erklären, daß die zu detektierenden Gase in der Praxis in Verbindung mit Feuchtigkeit auftreten. Durch die filternde Schicht kann die Feuchtigkeit zurückgehalten werden. Dadurch erhöht sich die Sensitivität, da diese sich als Quotient aus dem elektrischen Widerstand des Sensors in synthetischer Luft und dem elektrischen Widerstand des Sensors in dem zu detektierenden Gas berechnet. Zusätzlich sinkt die Empfindlichkeit auf NH₃, welches bei einem konventionellen Gassensor einen starken Meßeffekt bei trockener Luft aufweist. Da die feuchteundurchlässige Schicht die Bedeckung der Ga₂O₃-Oberfläche mit OH-Gruppen verhindert, läßt sich ein Sensor mit einer hohen Meßempfindlichkeit auf NH₃ herstellen.

Das Material, das als filternde Schicht eingesetzt werden soll, muß eine elektrische Leitfähigkeit aufweisen, die um mindestens den Faktor 10 geringer als die der gassensitiven Schicht ist. Weiterhin darf das Material nicht mit der gassensitiven Schicht reagieren und keine katalytische Umsetzung der Gase bewirken. Unporöses, amorphes SiO₂ ist ein Materialien mit den oben genannten Eigenschaften.

Bei Materialien mit filternden Eigenschaften, z. B. Fullerene, deren elektrische Leitfähigkeit zu hoch ist, oder Materialien, die mit der gassensitiven Schicht reagieren, wie z. B. durch Interdiffusion oder festkörperchemische Reaktionen, kann eine bis zu ca. 30 nm dicke, nichtleitende SiO₂-Schicht zwischen die gassensitive Schicht und die Filterschicht gebracht werden.

Die Betriebstemperatur des Gassensors hat zwischen 600 °C und 700 °C zu liegen. Bei einer niedrigeren Temperatur ist der elektrische Widerstand der gassensitiven Ga₂O₃-Schicht zu hoch. Bei einer höheren Temperatur geht die Sensitivität auf H₂ verloren.

Das Substrat SUB, auf dessen einer Seite die Heizanordnung H und auf dessen anderer Seite die Meßelektroden in Form einer Interdigitalstruktur I in Verbindung mit der gassensitiven Ga₂O₃-Schicht und der Filterschicht aufgebracht sind, kann aus einer Al₂O₃ (Aluminiumoxid) -Schicht und einer darüber liegenden elektrisch nichtleitenden SiO₂ (Siliziumdioxid) - Schicht bestehen. Die SiO₂-Schicht stellt gleichzeitig eine Diffusionssperrschicht dar.

### 2. Ausführungsform:

Bei der 2. Ausführungsform ist auf dem Substrat SUB eine 2 µm dicke Ga₂O₃ -Schicht aufgebracht (vgl. Figur 2). Über dieser Schicht ist eine gassensitive Metalloxidschicht aufgebracht. Die Dicke der gassensitiven Metalloxidschicht (auch als zweite Schicht bezeichnet) hängt vom verwendeten Metalloxid ab. In der Regel liegt sie zwischen 30 nm und 300 nm.

Der Gassensor wird wie folgt hergestellt:

Auf das Substrat SUB, das auf der einen Seite mit der Heizelektrodenstruktur H und auf der anderen Seite mit der Meßelektrodenstruktur I bedeckt ist, wird eine 2 µm dicke Galliumoxid-Schicht aufgebracht. Das Aufbringen erfolgt beispielsweise durch Sputtern. Anschließend erfolgt ein erster Temperprozeß bei einer Tempertemperatur ϑ_{T} zwischen 700 und 1200 °C. Dadurch wird die Kristallinität und die Stöchiometrie der Galliumoxid-Schicht verbessert. In einem nächsten Schritt wird auf die Oberfläche des Galliumoxids eine gassensitive Metalloxid-Schicht mit einer Schichtdicke zwischen 3 bis 300 nm aufgebracht. Diese Oberflächenmodifikation kann durch Kathodenzerstäubung CVD, Elektronenstrahlverdampfung, Molekularstrahlverfahren oder durch ein naßchemisches Verfahren erzeugt werden. Für die zweite Schicht kommen gassensitive Metalloxide, wie Titanoxid (TiO₂), Aluminiumvanadat (AlVO₄), V₂O₅, Wolframoxid (WO₃) oder Tantaloxid (TaO), zur Anwendung. Anschließend erfolgt ein zweiter Temperprozeß, der ca. 15 Stunden dauert und dessen Tempertemperatur ϑ_{T} je nach Betriebstemperatur ϑ_{B} zwischen 850°C und 1100°C liegt. Die Tempertemperatur ϑ_{T} ist grundsätzlich höher als die beabsichtigte Betriebstemperatur ϑ_{B} zu wählen. In der folgenden Tabelle ist für das jeweilige gassensitive Metalloxid die entsprechende Schichtstärke, die Tempertemperatur ϑ_{T}, die Betriebstemperatur ϑ_{B} und die jeweilige Gassensitivität des Sensors angegeben.

| **Metalloxid** | **Dicke [nm]** | **ϑ**_{**T**} **[°C]** | **ϑ**_{**B**} **[°C]** | **Sensitivität** |
|---|---|---|---|---|
| TiO₂ | 300 | 650 | 600 | Lösungsmittel |
| AlVO₄ | 300 | 750 | 700 | O₂-Sensor |
| AlVO₄ | 300 | 950 | 700-900 | nicht sensitiv = Referenzelement |
| AlVO₄ | 300 | 750 | 500 | NH₃ (Ammoniak) |
| AlVO₄ | 30 | 1050 | 1000 | nicht sensitiv auf Ethanol (C₂H₅OH), jedoch auf Methan (CH₄₎ |
| WO₃ | 30 | 350 | 300 | NO-Sensor |
| TaO | 300 | 750 | 700 | Ethanol-Sensor |

Eine nach dem ersten Temperprozeß aufgebrachte zweite Schicht aus Aluminiumvanadat wird durch den zweiten Temperprozeß zu Vanadiumpentoxid (V₂O₅).

Die zur Änderung der Leitfähigkeit beitragenen Elektronen stammen von der gassensitiven Metalloxidschicht (2. Schicht), nicht jedoch vom Galliumoxid. Die Elektronen bzw. das elektrische Feld wandern vom gassensitiven Metalloxid in das Galliumoxid und bewirken dort eine Leitfähigkeitsänderung. Es werden nicht die gassensitiven Eigenschaften des Galliumoxids, sondern die des als 2. Schicht aufgebrachten Metalloxids bzw. einer Kombination aus der zweiten Schicht und Galliumoxid ausgenutzt.

Bei einem konventionellen Galliumoxid-Sensor wird das zu messende Gas an der Oberfläche der Galliumoxid-Schicht chemiesorbiert oder es treten Oberflächenreaktionen an der halbleitenden Galliumoxidschicht auf und es kommt zu einem Elektronentransfer vom Adsorbat und Sensormaterial und damit zu einer Leitfähigkeitserhöhung.

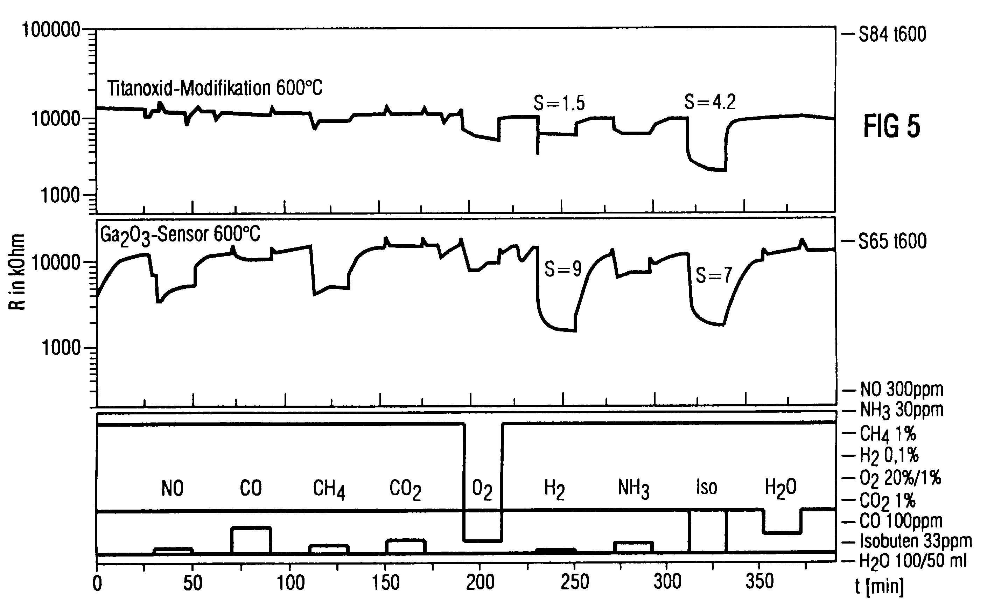

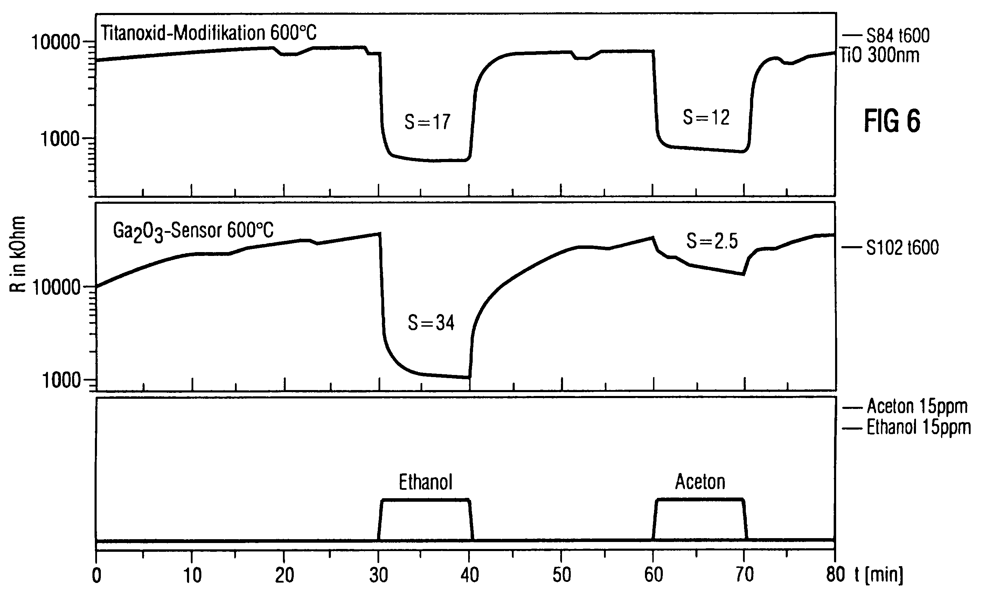

Wird die Oberfläche der Galliumoxid-Schicht durch das Aufbringen von einigen Atomlagen eines anderen gassensitiven Metalloxids verändert, so kommt es zu zwei positiven Effekten.
1. Es gelangen Elektronen, die von der Oberfläche durch den Gaseffekt generiert werden, in die Ga₂O₃-Schicht. Bei tiefen Temperaturen (600°C) sind dort nur sehr wenige Elektronen (10⁻¹³/cm³) vorhanden, wobei eine große Debye-Länge vorliegt. Elektronen, die in die Galliumoxid-Schicht gelangen, bewirken folglich eine große Änderung der Leitfähigkeit (ca. Faktor 10).
2. Auch die Chemiesorption bestimmter Gase, beispielsweise NO oder NH₃ wird durch das speziell ausgewählte gassensitive Metalloxid (2. Schicht) gefördert bzw. gehemmt. Dadurch kann gezielt die Empfindlichkeit (= Selektivität) für bestimmte Gase erhöht werden. In den Figuren 5 bis 16 werden jeweils oberflächenmodifizierter Sensor und ein unbeschichteter Ga₂O₃-Sensor (=konventioneller Sensor) gegenübergestellt. Die Darstellung der genannten Meßdiagramme entspricht der Darstellung der in den Figuren 3 und 4 gezeigten Meßdiagramme.

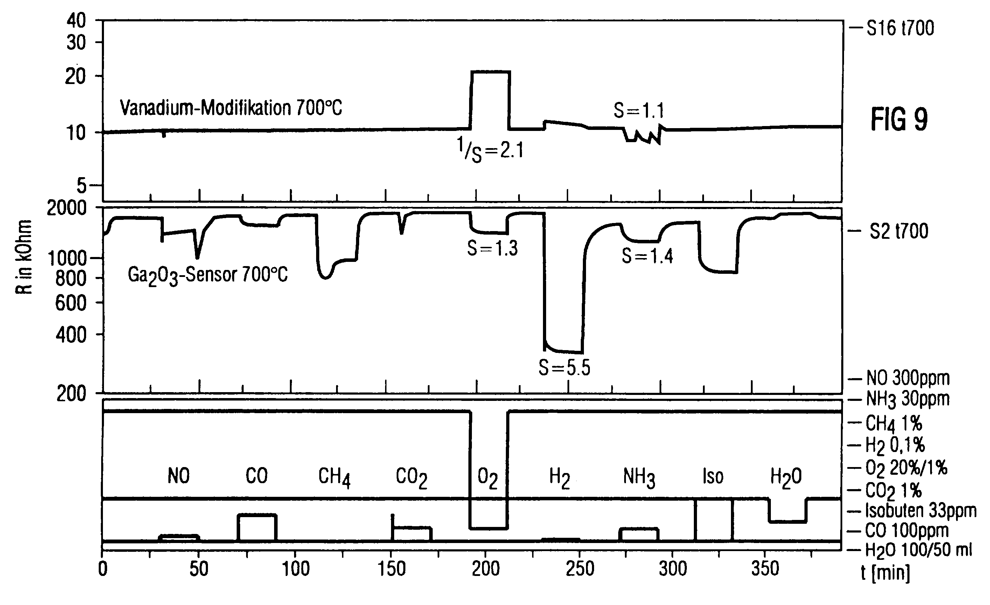

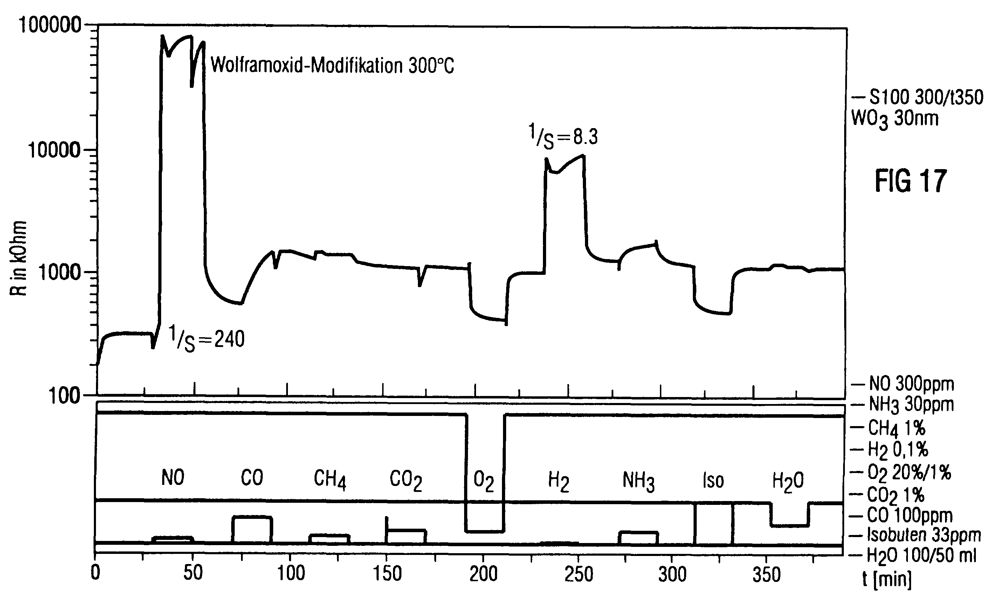

In den Figuren 17 und 9 ist der Widerstand des Sensors im Prüfgas kurzzeitig höher als der Widerstand des Sensors in sythetischer Luft. Um die Sensitivitäten an dieser Stelle besser vergleichen zu können, wird dort der Kehrwert der Sensitivität S angegeben.

Im folgenden sind Beispiele für selektive Sensoren, die eine gassensitive Metalloxidschicht aufweisen, angegeben.

### a) Lösungsmittel-Sensor:

In Figur 5 ist das gassensitive Verhalten eines mit einer Titanoxid-Schicht (Schichtdicke 300 nm) versehenen Gassensors im Vergleich zu einem konventionellen (unbeschichteten) Ga₂O₃-Sensor dargestellt. Der zweite Tempervorgang wurde bei einer Tempertemperatur ϑ_{T} = 650 °C durchgeführt. Die Betriebstemperatur liegt bei ϑ_{B} = 600 °C. Der erfindungsgemäße Sensor ist für Lösungsmittel geeignet. Die Sensitivität für Ethanol liegt bei 17, für Aceton bei 12 und für Isobuten bei 4,2. Bei allen anderen gemessenen Gasen liegt die Sensitivität unterhalb von 1,5. Der elektrische Widerstand des Sensors in synthetischer Luft liegt um ca. den Faktor 10 niedriger als bei dem konventionellen Galliumoxid-Sensor. Die Ansprechzeit des erfindungsgemäßen Gassensors konnten gegenüber dem konventionellen Galliumoxid-Sensor verringert werden.

### b) Ethanol-Sensor:

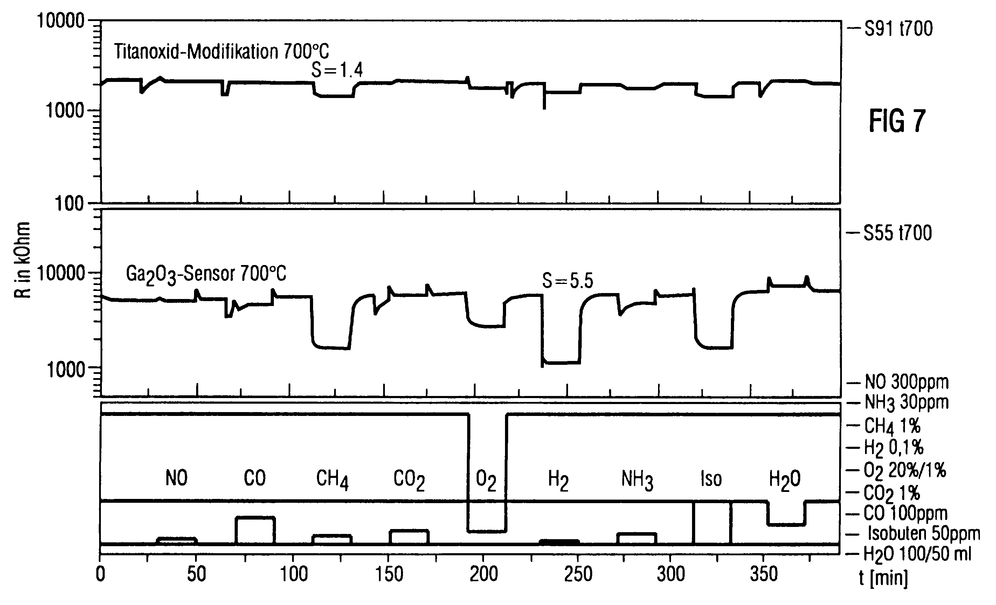

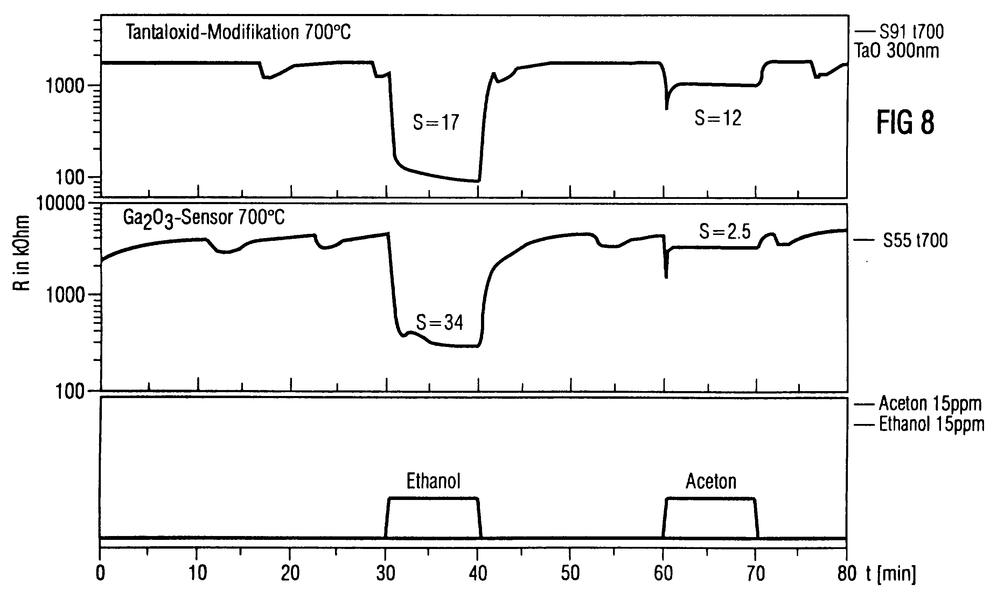

In den Figuren 7 und 8 ist das gassensitive Verhalten eines mit Tantaloxid beschichteten Gassensors (Schichtdicke 300 nm) im Vergleich zu einem unbehandelten Ga₂O₃-Sensor dargestellt. Der zweite Tempervorgang wurde bei ϑ_{T} = 750 °C durchgeführt. Die Betriebstemperatur liegt bei ϑ_{B} = 700 °C. Beide Sensoren reagieren vorwiegend auf Ethanol mit einer Sensitivität von S=19. Bei allen anderen Meßgasen liegt die Sensitivität des erfindungsgemäßen Sensors bei weniger als 1,7. Der elektrische Widerstand des erfindungsgemäßen Sensors in synthetischer Luft ist ca. um den Faktor 5 geringer als der des konventionellen Gassensors.

### c) Sauerstoff-Sensor:

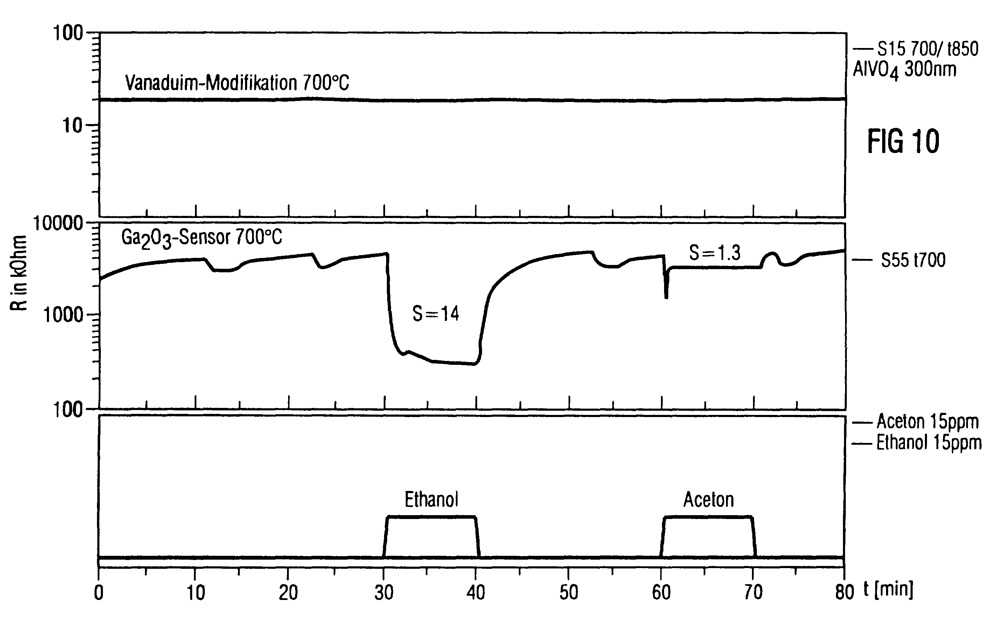

In den Figuren 9 und 10 ist das gassensitive Verhalten eines mit Aluminiumvanadat beschichteten Gassensors (Schichtdicke 300 nm) im Vergleich zu einem unbeschichteten Ga₂O₃-Sensor dargestellt. Der zweite Tempervorgang wurde bei ϑ_{T} = 750 °C durchgeführt. Die Betriebstemperatur liegt bei ϑ_{B} = 700 °C. Der erfindungsgemäße Sensor reagiert auf Sauerstoff (O₂) mit einer reziproken Sensitivität von 1/S = 2,1 und auf Ammoniak mit einer Sensitivität S = 1,1. Die Sensivität des erfindungsgemäßen Gassensors auf die anderen angegebenen Meßgase ist verschwindend gering. Der elektrische Widerstand des erfindungsgemäßen Sensors an synthetischer Luft ist ca. um den Faktor 100 kleiner als der des konventionellen Gassensors. Außerdem ist das Sensorsignal des erindungsgemäßen Gassensors sehr stabil und dessen Ansprechzeit klein.

### d) Ammoniak-Sensor:

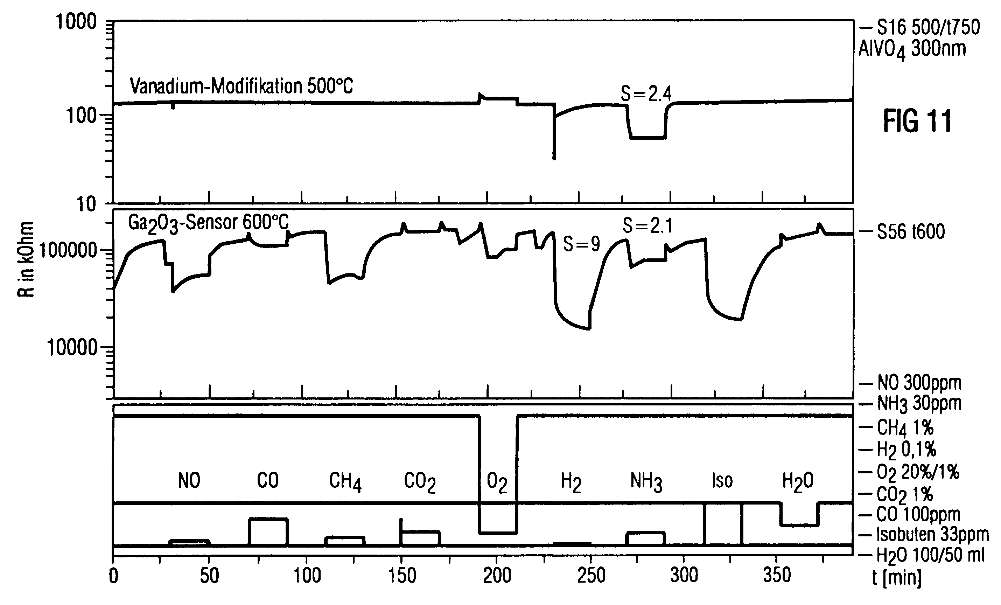

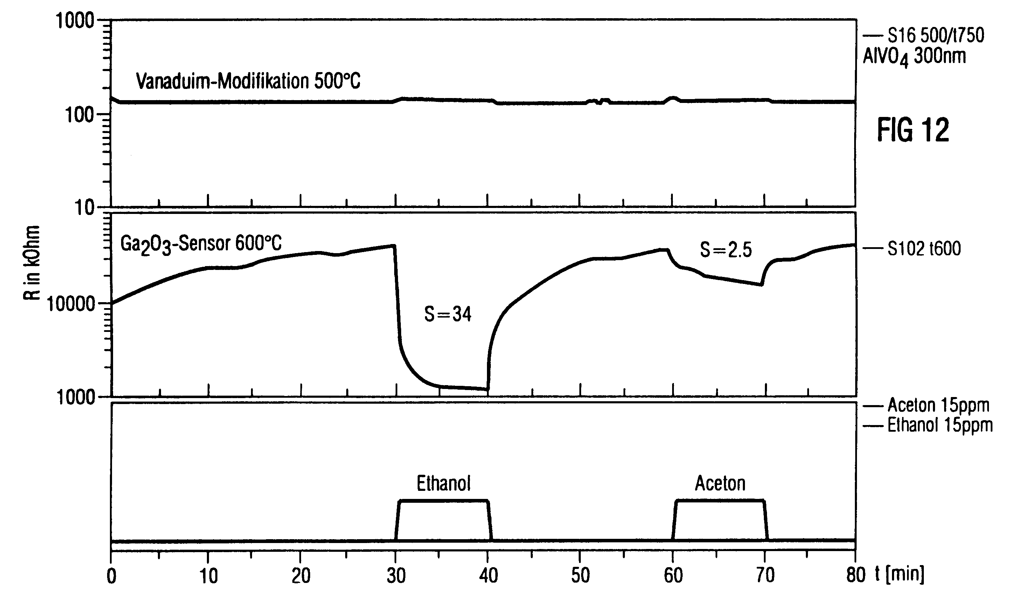

In den Figuren 11 und 12 ist das gassensitive Verhalten eines mit Aluminiumvanadat beschichteten Gassensors (Schichtdicke 300 nm) in Vergleich zu einem konventionellen Ga₂O₃-Sensor dargestellt. Die Tempertemperatur ϑ_{T} = 750 °C und die Betriebstemperatur (abweichend von dem unter Punkt c) angegebenen Sauerstoff -Sensor) liegt bei ϑ_{B} = 500 °C. Der erfindungsgemäße Sensor zeigt eine Sensitivität von S = 2,1 auf Ammoniak (NH₃) und eine sehr geringe Sensitivität auf Wasserstoff (H₂). Der elektrische Widerstand des erfindungsgemäßen Sensors in synthetischer Luft ist auch hier wesentlich geringer als bei einem konventionellen Ga₂O₃-Sensor. Das Sensorsignal des erfindungsgemäßen Sensors ist sehr stabil.

### e) Referenz-Sensor:

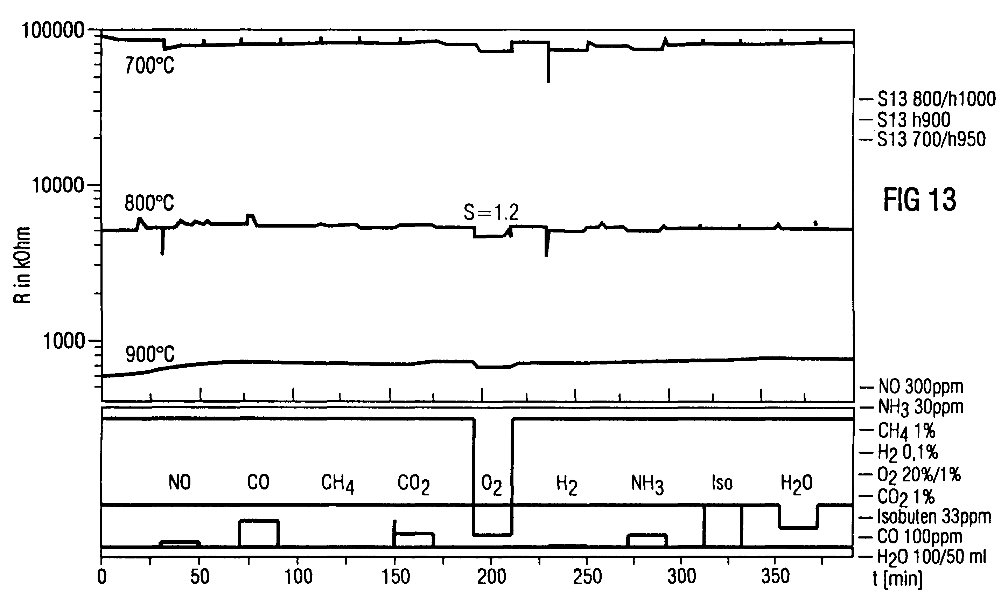

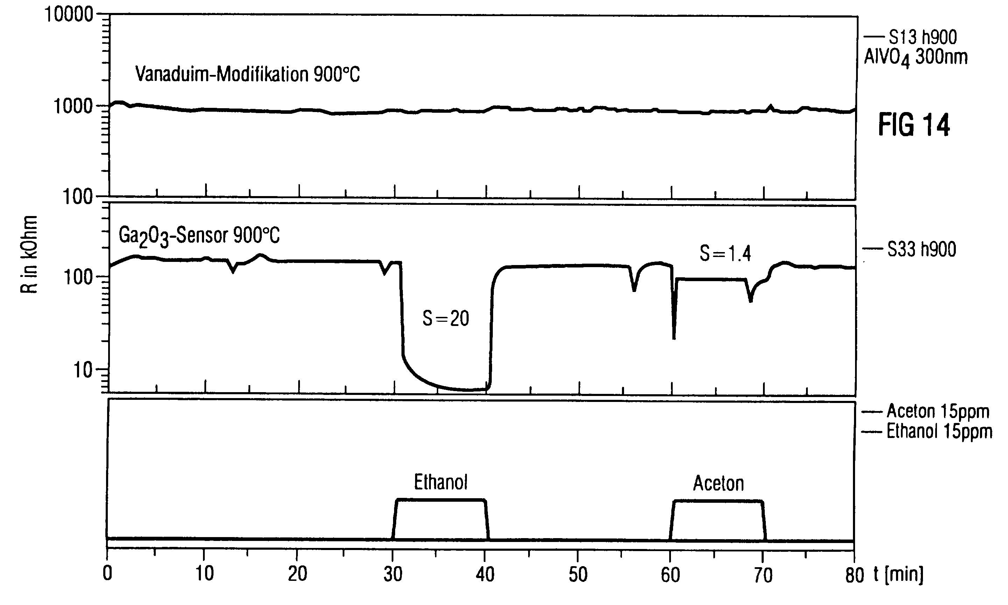

In den Figuren 13 und 14 ist das gassensitive Verhalten eines mit einer Aluminiumvanadat-Schicht versehenen Gassensors (Schichtdicke 300 nm) im Vergleich zu einem konventionellen Ga₂O₃-Sensor dargestellt. Die Tempertemperatur beträgt ϑ_{T} = 950 °C. Bei einer Betriebstemperatur ϑ_{B} zwischen 700 °C und 900 °C reagiert der erfindungsgemäße Sensor auf eine Veränderung des Sauerstoffgehalts lediglich mit einer Sensitivität S = 1,2. Die Sensitivitäten auf andere Meßgase ist erheblich geringer. Der angegebene erfindungsgemäße Sensor ist als gasinsensitives Referenzelement verwendbar.

### f) Methan-Sensor:

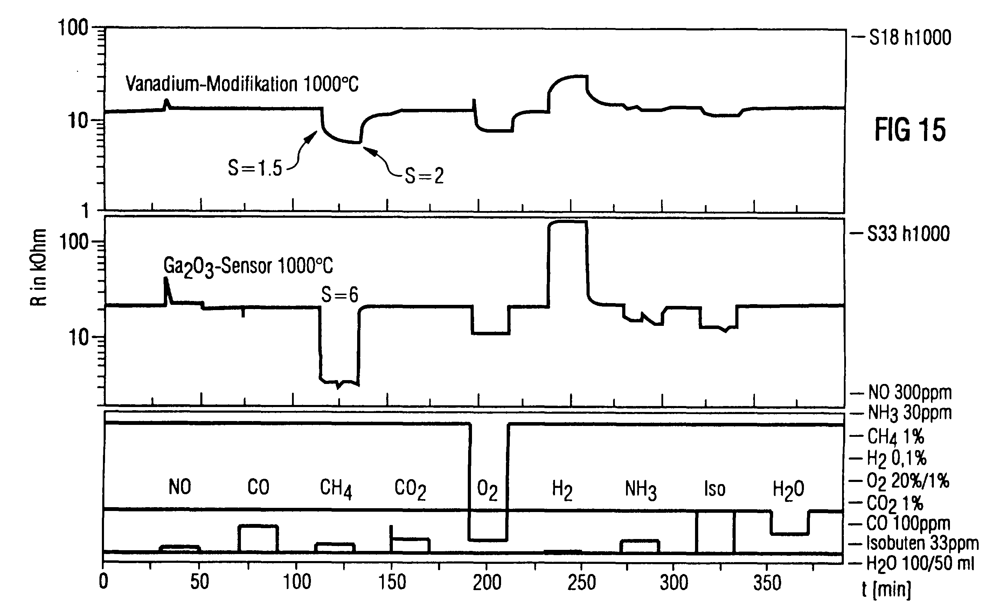

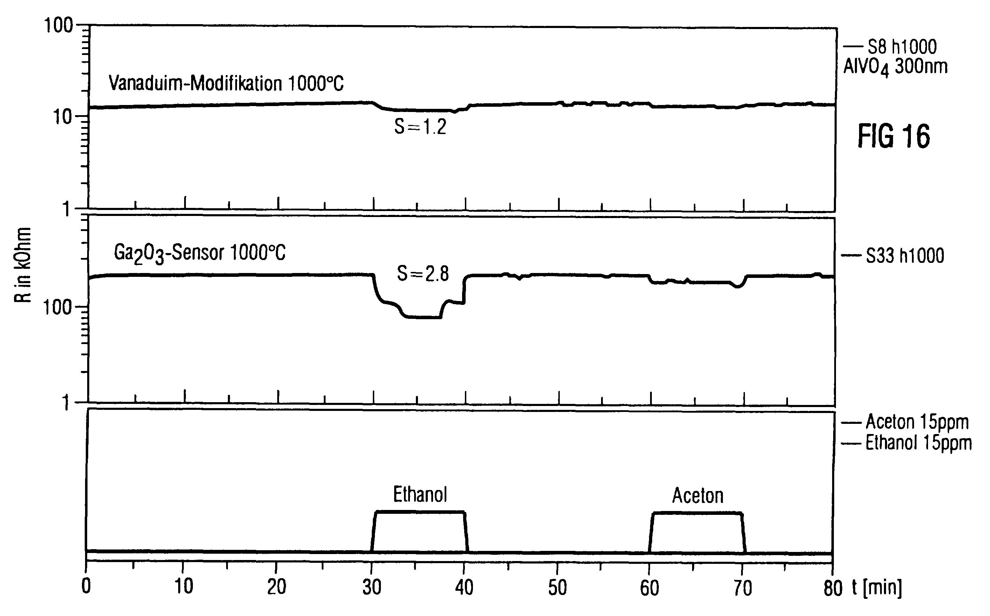

In den Figuren 15 und 16 ist das gassensitive Verhalten eines mit Aluminiumvanadat beschichteten Gassensors (Schichtdicke 30 nm) im Vergleich zu einem konventionellen Ga₂O₃-Sensor dargestellt. Die Tempertemperatur ϑ_{T} liegt bei 1050 °C, die Betriebstemperatur ϑ_{B} bei 1000 °C. Der Vorteil bei diesem erfindungsgemäßen Sensor liegt nicht in der Selektivität auf Methan, sondern in der Reduzierung der Sensitivität auf Ethanol, die für gewisse Anwendungen besonders störend wirken kann. Die Sensitivität auf Ethanol wurde um den Faktor 2, die auf Methan auf den Faktor 4 verringert, wenn man von gleichen Ansprechzeiten ausgeht. Die Methansensitivität wurde hierbei stärker reduziert. Selbst bei sehr viel höheren Konzentrationen des wenig stabilen Ethanols wird kaum eine höhere Sensitivität hervorgerufen, wohingegen bei Methan bereits die Konzentration vorliegt, die auch bei der technischen Anwendung des Sensors detektiert werden soll. Bei einem konventionellen Galliumoxid-Sensor steigt bei höheren Konzentrationen die Ethanol-Sensitivität um ein Vielfaches an.

### g) Stickstoffmonoxid-Sensor:

In den Figuren 15 und 16 ist das gassensitive Verhalten eines mit Wolframoxid beschichteten Gassensors (Schichtdicke 30 nm) im Vergleich zu einem unbehandelten Ga₂O₃-Sensor dargestellt. Die Tempertemperatur ϑ_{T} liegt bei 350 °C, die Betriebstemperatur ϑ_{B} bei 300 °C. Ein konventioneller Galliumoxid-Sensor kann wegen des hohen elektrischen Widerstands der sensitiven Schicht nicht unter 600 °C betrieben werden. Da bei 600 °C die höchste NO-Sensitivität vorhanden ist, wird diese Betriebstemperatur zum Vergleich herangezogen. Man erkennt die extrem hohe Sensitivität des erfindungsgemäßen Gassensors auf NO, die um ca. den Faktor 100 größer ist (1/S mit S verglichen). Der elektrische Widerstand des erfindungsgemäßen Sensors in Luft liegt bei ca. 1 Megaohm und ist somit ebenfalls um den Faktor 100 geringer als der des konventionellen Galliumoxidgas-Sensors.

Der erfindungegemäße Sensor wird vorteilhafterweise in kostengünstiger und mikrosystemkompatibler Planartechnologie hergestellt. Dieser Technologie ist, von der Reproduzierbarkeit der Herstelltechnologie aus gesehen, der Vorzug gegenüber bulk-keramischen Aufbauten zu geben.

Durch die Aufbringung einer gassensitiven Metalloxid-Schicht konnte die Gassensitivität in hohem Maße beeinflußt werden. Die Sensitivitäten auf bestimmte Zielgase konnte vergrößert, die Querempfindlichkeiten auf interferrierende Gase verringert oder zum Teil ganz beseitigt werden. Die sehr hohe Ethanolempfindlichkeit eines konventionellen Galliumoxidgas-Sensors konnte beim erfindungsgemäßen Sensor stark verringert werden, wobei die Methansensitivität nur gering abnahm (vgl. Figur 16).

Das gasinsensitive Referenzelement gemäß Figur 13 und 14 kann zur Elimination von Temperaturschwankungen verwendet werden. Dadurch ist eine Regelung der Temperatur des Sensors nicht mehr notwendig. Hierzu wird auf die Anmeldung P 44 32 729.3 verwiesen. Dort ist ein Gassensor angegeben, der eine gas- und temperaturabhängige Anordnung und eine in unmittelbarer Nähe dazu angeordnete, lediglich temperaturabhängige Anordnung aufweist. Durch Differenz- oder Verhältnisbildung der Ausgangssignale der beiden Anordnungen ist eine von Temperaturschwankungen unabhängige und nur mehr gasabhängige Meßgröße zu erhalten. Die erste Schicht aus Ga₂O₃ stellt die stabile elektrische Grundleitfähigkeit zur Verfügung.

## Patentansprüche

1. Gassensor
- bei dem eine gassensitive Schicht vorgesehen ist, die Ga₂O₃ aufweist,
- bei dem über der gassensitiven Schicht eine Filterschicht vorgesehen ist, die SiO₂ aufweist.

2. Gassensor nach Anspruch 1,
bei dem die Filterschicht 300 nm bis 500 nm dick ist.

3. Gassensor nach Anspruch 1 oder 2,
bei dem die Betriebstemperatur zwischen 600 °C und 700 °C liegt.

4. Verfahren zur Herstellung eines Gassensors nach einem der Ansprüche 1 bis 3,
bei dem die Filterschicht durch Kathodenzerstäubung, CVD, Elektronenstrahlverdampfung, Molekularstrahlverdampfung oder ein naßchemisches Verfahren aufgebracht wird.

5. Gassensor,
- bei dem eine erste Metalloxidschicht vorgesehen ist, die Ga₂O₃ aufweist,
- bei dem über der ersten Schicht eine zweite Schicht vorgesehen ist, die ein gassensitives Metalloxid aufweist.

6. Gassensor nach Anspruch 5,
bei dem das gassensitive Metalloxid der zweiten Schicht TiO₂, V₂O₅, WO₃ oder TaO ist.

7. Gassensor nach Anspruch 5,
bei dem in der zweiten Schicht MgO, ZrO₂ und/oder BeO enthalten ist.

8. Verfahren zur Herstellung eines Gassensors nach Anspruch 5,
- bei dem auf ein mit einer Elektrodenstruktur versehenens Substrat (SUB) Ga₂O₃ aufgebracht wird,
- bei dem ein erster Tempervorgang durchgeführt wird,
- bei dem auf das Ga₂O₃ eine Schicht aus TiO₂, WO₃, TaO oder AlVO₄ aufgebracht wird,
- bei dem ein zweiter Tempervorgang durchgeführt wird.
